# EUROPEAN PATENT APPLICATION

(11) **EP 4 318 498 A1**
(43) Date of publication of application: **07.02.2024**
(21) Application number: 22775850.5
(22) Date of filing: 25.03.2022
(51) Int. Cl.: G16H 70/40

(54) **DOSING SUBJECT MANAGEMENT SYSTEM, MANAGEMENT METHOD, MANAGEMENT CONTROL DEVICE, TERMINAL DEVICE, AND PROGRAM STORAGE MEDIUM**

(30) Priority: 26.03.2021 JP 2021052634
(71) Applicant: NTT Communications Corporation, Tokyo 100-8019 (JP)
(72) Inventor: TSUNEKAWA, Satoshi, Tokyo 100-8019 (JP); KONAGAYA, Takahito, Tokyo 100-8019 (JP); SAKURAI, Yoichi, Tokyo 100-8019 (JP); IZUMI, Hiroaki, Tokyo 100-8019 (JP); TABATA, Masaaki, Tokyo 100-8019 (JP)
(74) Representative: Gramm, Lins & Partner Patent- und Rechtsanwälte PartGmbB
(86) International application number: PCT/JP2022/014630
(87) International publication number: WO 2022/203069

(57) **Abstract**

Management of post-medication conditions for a wider range of medication target persons who have been administered medications is enabled. A management control apparatus acquires and manages, from a terminal device used by a medication target person, post-medication information discretionarily input by the medication target person after the medication, and post-medication information input for a designated report item as medicated person notebook information and report information for a medication manager, respectively. Then, by selectively using the medicated person notebook information and the report information for the medication manager, handling processing is executed according to a change in post-medication condition of the medication target person.

## Description

### FIELD

Embodiments described herein relate generally to, for example, a medication target person management system, a management method, a management control apparatus, a terminal device, and a program storage medium for managing a condition of a patient who has received medication.

### BACKGROUND

In the medical or pharmaceutical field, so-called clinical trials are carried out in which the conditions of patients after medication are followed and researched. In conventional clinical trials, for example, inpatients or outpatients are subjects, and generally, a clinical trial coordinator extracts clinical trial subjects on the basis of medical records or the like of the patients to conduct the clinical trials. For this reason, there is a problem in that much labor and time are required for the work for extracting the clinical trial subjects, and failure to extract the patients who should have been subjects is likely to occur.

Therefore, there has been proposed a system in which prescription and dispensing information of inpatients or outpatients is registered in a database, and the registered prescription and dispensing information is data-collated with clinical trial information indicating a preset clinical trial standard by an information processing apparatus, thereby screening clinical trial subjects and constructing a database of suitable patients for a clinical trial (for example, refer to Patent Literature 1).

### CITATION LIST

### PATENT LITERATURE

Patent Literature 1: Japanese Patent No. 6338957

### SUMMARY

### TECHNICAL PROBLEM

However, in the system as proposed above, the subjects of clinical trials are limited to patients whose medical care information such as medical records or prescriptions is managed by medical institutions, pharmacies, or the like, such as inpatients and outpatients. Since the number of clinical trials is small, there is a problem in that subjects of clinical trials cannot be extracted from a wider range of patient groups.

In particular, for example, in a case where follow-up research after vaccination is to be performed on vaccination subjects, many healthy persons are included in the vaccination subjects, and medical care information such as medical records or prescriptions is not managed in medical institutions, pharmacies, or the like for those vaccination subjects in many cases. Therefore, it is difficult to perform follow-up research after vaccination with the proposed system described above.

The present invention has been made in view of the above circumstances, and in one aspect, an object of the present invention is to provide a technique that enables management of post-medication conditions for a wider range of medication target persons who have been administered medications.

### SOLUTION TO PROBLEM

In order to solve the above problem, one aspect of the present invention relates to a medication target person management system for managing a post-medication condition of a medication target person, the medication target person management system including a terminal device configured to be used by the medication target person and a management control apparatus capable of communicating information data with the terminal device via a network.

The terminal device has a function of transmitting, to the management control apparatus via the network, first medication management information including the post-medication condition of the medication target person input for a discretionary item, and a function of transmitting, to the management control apparatus via the network, second medication management information including the post-medication condition of the medication target person input for a report item designated in advance.

On the other hand, the management control apparatus includes a first storage section in which a storage area for storing medicated person notebook information corresponding to the medication target person is set, and a second storage section in which a storage area for storing report information for a medication manager corresponding to the medication target person is set. The management control apparatus further includes: a first acquisition processing section configured to acquire the first medication management information from the terminal device via the network, associate the acquired first medication management information with identification information of the medication target person, and cause the first storage section to store the first medication management information as the medicated person notebook information; a second acquisition processing section.configured to acquire the second medication management information from the terminal device via the network, associate the acquired second medication management information with the identification information of the medication target person, and cause the second storage section to store the second medication management information as the report information; and a post-medication handling processing section configured to selectively use the stored first medication management information and the second medication management information to execute handling processing according to a change in the post-medication condition for the medication target person.

According to one aspect of the present invention, in the management control apparatus, post-medication information discretionarily input by the medication target person after the medication and post-medication information input for a designated report item are acquired and managed from a terminal device respectively as medicated person notebook information and report information for a medication manager, and the medicated person notebook information and the report information for the medication manager are selectively used, and handling processing is executed according to a change in post-medication condition of the medication target person.

Therefore, for example, even for a medication target person whose medical record or the like is not managed in a medical institution or the like, information after medication can be collected and managed by the management control apparatus by using the terminal device of the medication target person. Accordingly, it is possible to manage conditions of a wider range of medication target persons after the vaccination. In addition, by selectively using the medicated person notebook information and the report information for the medication manager to deal with a change in post-medication condition of the medication target person, it is possible to perform appropriate handling processing in consideration of the properties of both pieces of information.

### ADVANTAGEOUS EFFECTS OF INVENTION

That is, according to one aspect of the present invention, it is possible to provide a technique that enables management of post-medication conditions for a wider range of medication target persons who have been administered medications.

### BRIEF DESCRIPTION OF THE DRAWINGS

FIG. 1 is a diagram showing an overall configuration of a medication target person management system according to a first embodiment of the present invention.
FIG. 2 is a block diagram showing a hardware configuration of a vaccinated person terminal according to the first embodiment of the present invention.
FIG. 3 is a block diagram showing an example of a software configuration of the vaccinated person terminal shown in FIG. 2.
FIG. 4 is a block diagram showing a hardware configuration of a management control apparatus according to the first embodiment of the present invention.
FIG. 5 is a block diagram showing an example of a software configuration of the management control apparatus shown in FIG. 4.
FIG. 6 is a flowchart showing procedures and details of pre-vaccination to vaccination processing executed by the vaccinated person terminal shown in FIG. 3.
FIG. 7 is a flowchart showing procedures and details of post-vaccination processing executed by the vaccinated person terminal shown in FIG. 3.
FIG. 8 is a flowchart showing an example of procedures and details of pre-vaccination to vaccination processing executed by the management control apparatus shown in FIG. 5.
FIG. 9 is a flowchart showing an example of procedures and details of post-vaccination processing executed by the management control apparatus shown in FIG. 5.
FIG. 10 is a flowchart showing an example of procedures and details of on-line medical care support processing of the procedures shown in FIG. 9.
FIG. 11 is a sequence diagram showing a flow of information data in pre-vaccination to vaccination processing among the management control apparatus shown in FIG. 3, its peripheral systems, and the vaccinated person terminal.
FIG. 12 is a sequence diagram showing a flow of information data in post-vaccination processing among the management control apparatus shown in FIG. 3, its peripheral systems, and the vaccinated person terminal.
FIG. 13 is a sequence diagram showing a flow of information data in a case of ending a PMS report in post-vaccination processing among the management control apparatus shown in FIG. 3, its peripheral systems, and the vaccinated person terminal.

### DETAILED DESCRIPTION

Embodiments of the present invention will be described below with reference to the drawings.

### First Embodiment

### (Configuration Example)

### (1) System

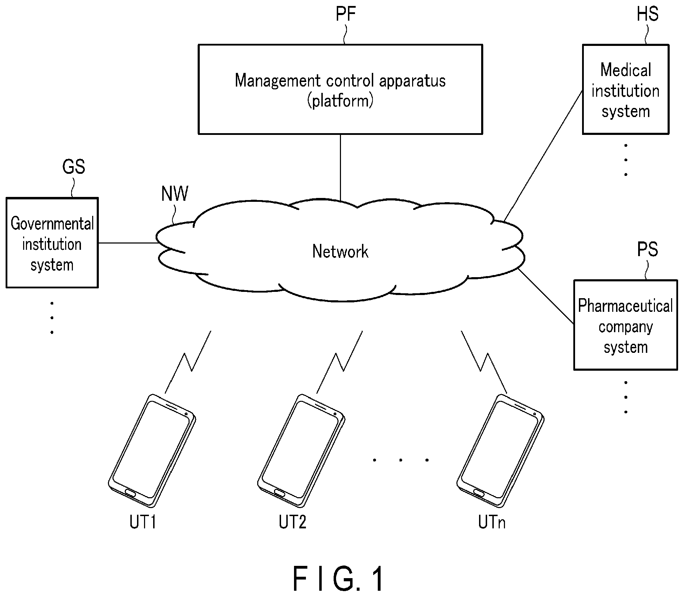

FIG. 1 is a diagram showing an example of an overall configuration of a medication target person management system according to a first embodiment of the present invention. PF indicates a management control apparatus which is a core of the system. The management control apparatus PF is hereinafter also referred to as a platform.

The platform PF can perform data transmission with vaccinated person terminals UT1 to UTn (hereinafter collectively referred to as UT) used by vaccinated persons via a network NW. Further, the platform PF can perform data transmission with a system HS operated by a medical institution, a system PS operated by a pharmaceutical company, and a system GS operated by a governmental institution such as a local government via the network NW.

Each of the system HS operated by the medical institution, the system PS operated by the pharmaceutical company, and the system GS operated by the governmental institution includes a server computer including a database and a management terminal used by a person in charge of management of each system. The person in charge of each of the medical institution, the pharmaceutical company, and the governmental institution uses the management terminal to transmit and receive information data to and from the platform PF or the vaccinated person terminal UT.

The network NW includes, for example, a wide area network having the Internet as a core and an access network for accessing the wide area network. As the access network, a wired or wireless public communication network, a wired or wireless local area network (LAN), and a cable television (CATV) network are used.

### (2) Apparatus

### (2-1) Vaccinated Person Terminal UT

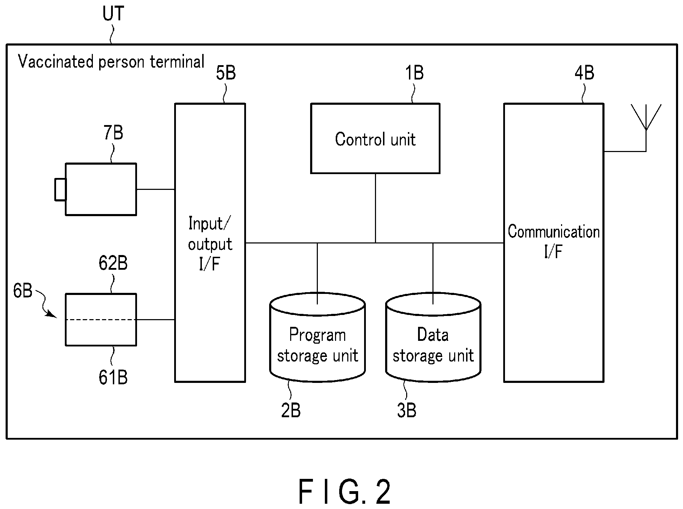

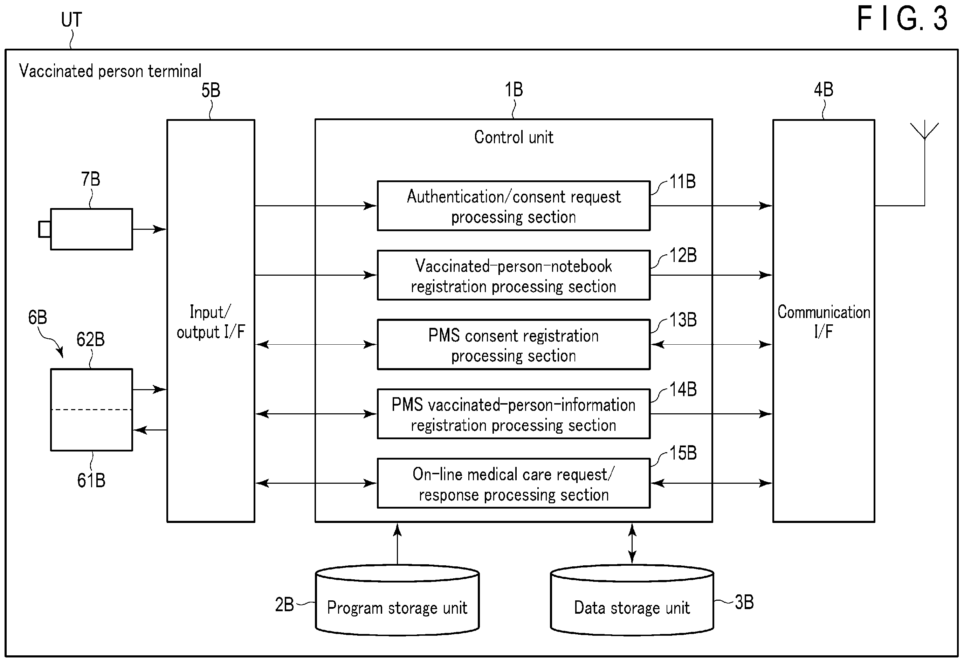

FIGS. 2 and 3 are block diagrams respectively showing examples of a hardware configuration and a software configuration of a vaccinated person terminal UT.

The vaccinated person terminal UT is composed of, for example, a general-purpose smartphone including a browser and a communication application for transferring information data using email, a social network system (SNS), a short message service (SMS), or the like. As the vaccinated person terminal UT, a tablet terminal, a notebook personal computer, or the like may be used as long as it has a similar function.

The vaccinated person terminal UT includes a control unit 1B using a hardware processor such as a central processing unit (CPU). The control unit 1B is connected to a storage unit including a program storage unit 2B and a data storage unit 3B, a communication interface ("interface" is hereinafter referred to as "I/F") 4B, and an input/output I/F 5B.

The communication I/F 4B performs data transmission with respect to the platform PF using a communication protocol defined by the network NW under the control of the control unit 1B. As the communication protocol, for example, Transmission Control Protocol/Internet Protocol (TCP/IP) is used.

The input/output I/F 5B is connected to an input/output device 6B and a camera 7B. The input/output device 6B is composed of, for example, a display section 61B using liquid crystal or organic EL, and an input section 62B using a touch-type input sheet of a pressure-sensitive type or a capacitance type disposed on a display screen of the display section 61B. As the input/output device 6B, other devices such as a keyboard and a mouse may be used.

The input section 62B is used by the vaccinated person to input, for example, basic information and consent information of the vaccinated person, medical inquiry information, and information indicating the condition of the vaccinated person after the vaccination, in addition to normal terminal operations. The basic information includes attribute information such as the name, age, address, contact information, and the like of the vaccinated person. The information indicating the condition after the vaccination includes discretionary information indicating the physical condition and the like of the vaccinated person to be registered by the person him/herself in a vaccinated person notebook, which will be described later, and information for reporting the condition after the vaccination by the vaccinated person with respect to a designated item in response to a request for execution of post-marketing surveillance (PMS) from a pharmaceutical company.

The display section 61B is used for displaying, in addition to various kinds of information necessary for normal operation of the terminal, various kinds of information data input as described above, vaccine information distributed from the pharmaceutical company or the governmental institution, image information of E-learning for conducting PMS, image data sent from a doctor terminal in on-line medical care, and the like.

The camera 7B is used for optically reading, for example, information written on a vaccination ticket sent before vaccination from a governmental institution such as a local government to a vaccination target person, or information written on a vaccination certification seal or a vaccination completion certificate issued by a medical worker or the like at a vaccination site in a vaccination project conducted by the nation based on the Preventive Vaccination Act. The information on the vaccination ticket and the vaccination certification seal are generally represented by a code data, such as a bar code or a QR code (registered trademark), whereas the vaccination completion certificate is generally written in plain text so that the information can be directly confirmed by the vaccinated person. In a case where the consent information and the medical inquiry information are written on the sheet, the camera 7B may be used to optically read the consent information and the medical inquiry information.

In addition, the input/output I/F 5B may be connected to a global positioning system (GPS) sensor for measuring the position of the vaccinated person or a vital sensor for measuring biological information such as the body temperature, blood pressure, and heart rate of the vaccinated person.

As a storage medium, the program storage unit 2B is configured, for example, to be a combination of a nonvolatile memory, such as a solid state drive (SSD), for which writing and reading can be performed at any time, and a nonvolatile memory, such as a read only memory (ROM). In addition to middleware such as an operating system (OS), the program storage unit 2B stores application programs necessary to execute various controlling processes relating to the first embodiment. Hereinafter, the OS and the application programs are collectively referred to as programs.

As a storage medium, the data storage unit 3B is, for example, a combination of a nonvolatile memory, such as an SSD, for which writing and reading can be performed at any time, and a volatile memory, such as a random access memory (RAM), and is used to store information data received from the platform PF and various types of information data input by the vaccinated person.

The control unit 1B includes, as processing functions related to the present invention, an authentication/consent request processing section 11B, a vaccinated-person-notebook registration processing section 12B, a PMS consent registration processing section 13B, a PMS vaccinated-person-information registration processing section 14B, and an on-line medical care request/response processing section 15B. Each of these processing sections 11B to 15B is realized by causing the hardware processor of the control unit 1B to execute a program stored in the program storage unit 2B.

The authentication/consent request processing section 11B executes various authentication procedures and consent procedures with respect to the platform PF when registering a use of the vaccinated person terminal UT with respect to the platform PF.

The vaccinated-person-notebook registration processing section 12B fetches, via the input/output I/F 5B, the basic information of the vaccinated person, the written information of the vaccination ticket, the medical inquiry information, the written information of the vaccination certification seal or the vaccination completion certificate, and the post-vaccination information discretionarily representing the condition of the vaccinated person after the vaccination, which are input by the vaccinated person at the input section 62B. Then, the fetched information data described above are transmitted from the communication I/F 4B to the platform PF so that the fetched information data is registered in a vaccinated-person-notebook storage section provided in the platform PF.

In a case where a request for cooperation with the PMS and a request for execution are sent from the pharmaceutical company system PS via the platform PF, the PMS consent registration processing section 13B performs processing of returning information indicating whether the vaccinated person has consented or not input by the vaccinated person in response to these requests from the communication I/F 4B to the platform PF.

The PMS vaccinated-person-information registration processing section 14B fetches, via the input/output I/F 5B, PMS report information representing the condition of the vaccinated person itself after the vaccination input by the vaccinated person through the input section 62B in accordance with the designated items of the PMS transmitted in advance from the pharmaceutical company system PS. Then, in order to register the fetched PMS report information in a PMS vaccinated-person-information storage section of the platform PF, the PMS vaccinated-person-information registration processing section 14B performs processing of transmission from the communication I/F 4B to the platform PF.

In a case where the vaccinated person inputs a request for receiving on-line medical care consultation, the on-line medical care request/response processing section 15B transmits a request for execution of on-line medical care from the communication I/F 4B to the platform PF. Upon connection to a doctor terminal by a communication link for on-line medical care, transmission/reception control of information such as image data and voice data for on-line medical care is performed.

### (2-2) Platform PF

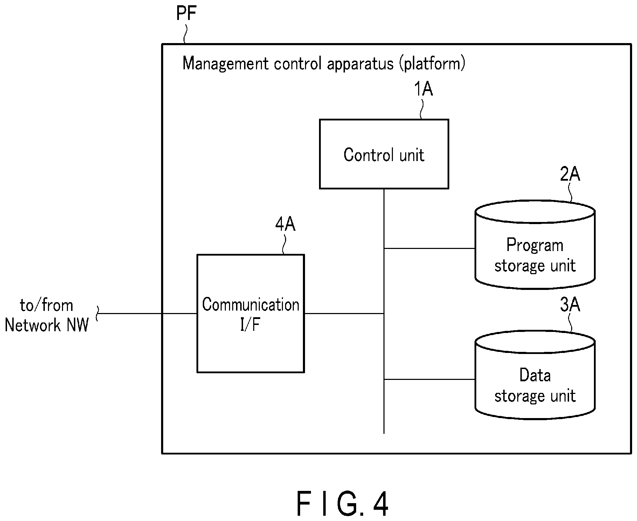

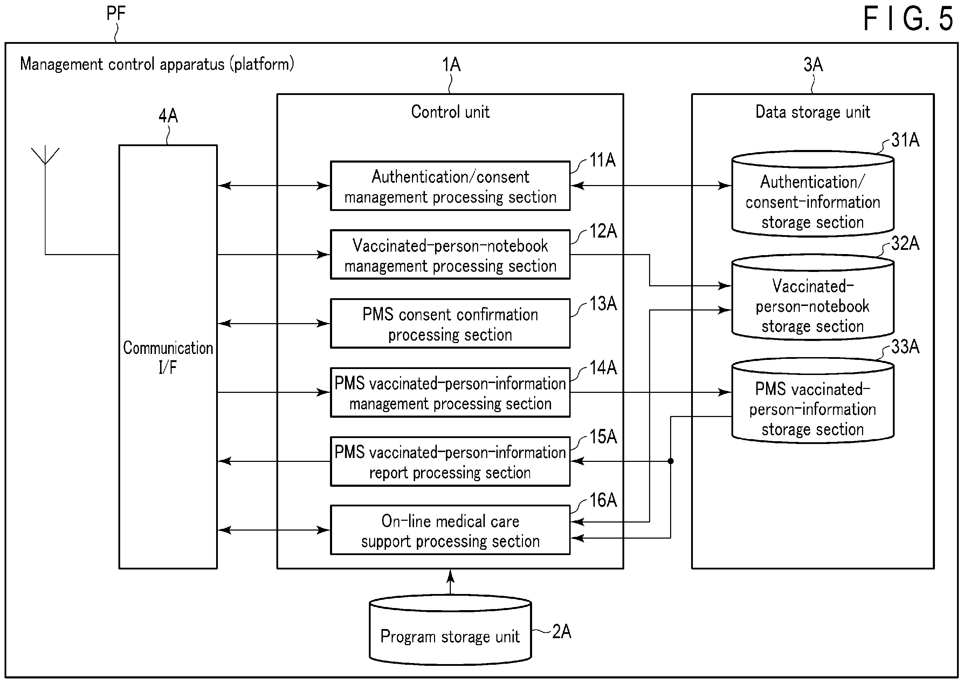

FIGS. 4 and 5 are block diagrams respectively showing examples of a hardware configuration and a software configuration of the platform PF, respectively.

The platform PF includes, for example, a server computer provided in a cloud, and includes a control unit 1A using a hardware processor such as a CPU. The control unit 1A is connected to a storage unit including a program storage unit 2A and a data storage unit 3A, and a communication I/F 4A. In this example, a case where the platform PF is realized by one server computer is described as an example. However, the platform PF may be configured such that a plurality of functions are separately performed by a plurality of server computers.

Under the control of the control unit 1A, the communication I/F 4A uses a communication protocol defined by the network NW to transmit and receive information data among the vaccinated person terminal UT, the governmental institution system GS, the pharmaceutical company system PS, and the medical institution system HS.

As a storage medium, the program storage unit 2A is configured, for example, to be a combination of a nonvolatile memory such as a hard disk drive (HDD), an SSD, or the like for which writing and reading can be performed at any time, and a nonvolatile memory such as a ROM. In addition to middleware such as an OS, the program storage section 2A stores programs necessary to execute various controlling processes relating to the first embodiment of the present invention.

As a storage medium, the data storage unit 3A is, for example, a combination of a non-volatile memory such as an HDD, an SSD, or the like for which writing and reading can be performed at any time, and a volatile memory such as a RAM. The data storage unit 3A includes, as main storage sections necessary to implement the first embodiment of the present invention, an authentication/consent-information storage section 31A, a vaccinated-person-notebook storage section 32A, and a PMS vaccinated-person-information storage section 33A.

The authentication/consent-information storage section 31A is used to store various authentication information and consent information generated or received by the authentication procedure and consent procedure performed with respect to the vaccinated person terminal UT. The authentication information includes an account (personal ID) owned by the vaccinated person to use, for example, a communication carrier or an SNS, and a vaccinated person ID used by the vaccinated person to use the platform PF, and these IDs are stored so as to be able to associate with each other.

The vaccinated-person-notebook storage section 32A is used to store the basic information of the vaccinated person, the written information of the vaccination ticket, the medical inquiry information, the written information of the vaccination certification seal or the vaccination completion certificate, discretionary information indicating a physical condition after the vaccination, and the like, which are transmitted from the vaccinated person terminal UT.

The PMS vaccinated-person-information storage section 33A has a PMS information storage area prepared corresponding to each vaccinated person who has consented to the request for cooperation with the PMS, and is used for storing the PMS report information sent from the corresponding vaccinated person in the PMS information storage area.

The control unit 1A includes, as main processing functions necessary for implementing the first embodiment of the present invention, an authentication/consent management processing section 11A, a vaccinated-person-notebook management processing section 12A, a PMS consent confirmation processing section 13A, a PMS vaccinated-person-information management processing section 14A, a PMS vaccinated-person-information report processing section 15A, and an on-line medical care support processing section 16A. Each of these processing sections 11A to 16A is realized by causing the hardware processor of the control unit 1A to execute a program stored in the program storage unit 2A.

The authentication/consent management processing section 11A executes various authentication procedures and consent procedures necessary for the vaccinated person to use the platform PF with respect to the vaccinated person terminal UT. The authentication procedures include, for example, setting up an account for the vaccinated person to use the vaccinated person notebook and generating of authentication information necessary for the subsequent use of the vaccinated person notebook. Japanese Public Key Infrastructure (JPKI) may be used for identity authentication of the vaccinated person. The consent procedures include, in addition to the consent confirmation of the vaccinated person with respect to the terms of use of the platform PF, the consent confirmation with respect to the provision of the information of the vaccinated person notebook of the vaccinated person to the pharmaceutical company or the governmental institution such as the local government, and the consent confirmation with respect to the distribution of the vaccine information or the like by the pharmaceutical company or the governmental institution to the vaccinated person.

The vaccinated-person-notebook management processing section 12A receives, via the communication I/F 4A, the basic information of the vaccinated person, the written information of the vaccination ticket, the medical inquiry information, the written information of the vaccination certification seal or the vaccination completion certificate, and discretionary information indicating the physical condition after the vaccination, which are sent from the vaccinated person terminal UT. Then, the vaccinated-person-notebook management processing section 12A performs processing of storing the received information data in association with the vaccinated person ID in a vaccinated-person-notebook storage area corresponding to the vaccinated person of the request source in the vaccinated-person-notebook storage section 32A.

In response to a request for cooperation with the PMS and a request for execution of the PMS sent from the pharmaceutical company system PS to the vaccinated person, the PMS consent confirmation processing section 13A performs processing of confirming the consent to the request for cooperation with the PMS and the request for execution of the PMS with respect to the vaccinated person terminal UT.

The PMS vaccinated-person-information management processing section 14A sets a storage area for PMS report information in the PMS vaccinated-person-information storage section 33A for each vaccinated person who has consented to the request for cooperation and the request for execution of the PMS. Thereafter, in a case where the PMS report information is transmitted from the vaccinated person terminal UT, the PMS vaccinated-person-information management processing section 14A receives the report information via the communication I/F 4A, and causes the received PMS report information to be stored in the storage area for the PMS report information corresponding to the vaccinated person in the PMS vaccinated-person-information storage section 33A.

For example, each time new PMS report information is sent from the vaccinated person terminal UT, or in a case where the PMS report information includes information about suspected side reactions, the PMS vaccinated-person-information report processing section 15A reads the PMS report information from the corresponding storage area of the PMS vaccinated-person-information storage section 33A, and transmits the read PMS report information from the communication I/F 4A to the pharmaceutical company system PS.

The on-line medical care support processing section 16A selects a medical institution that addresses on-line medical care, in a case where a request for a health consultation or a request for on-line medical care consultation after vaccination is sent from the vaccinated person terminal UT. Then, the doctor terminal of the selected medical institution and the vaccinated person terminal of the request source are connected by a communication link to enable transmission of information data for on-line medical care. An example of this processing will be described in an operation example.

### (Operation Example)

Next, an operation example of the system including the platform PF configured as described above will be described.

### (1) Processing before and at the time of vaccination

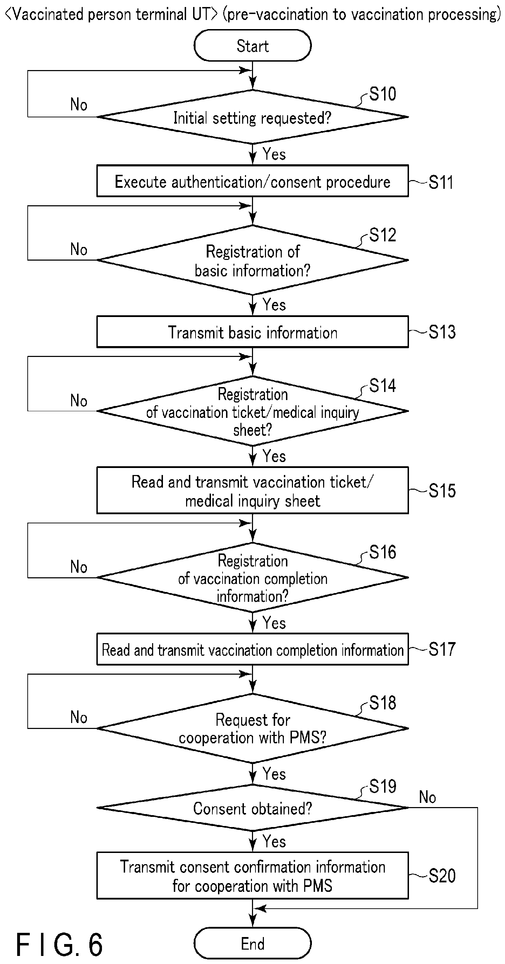

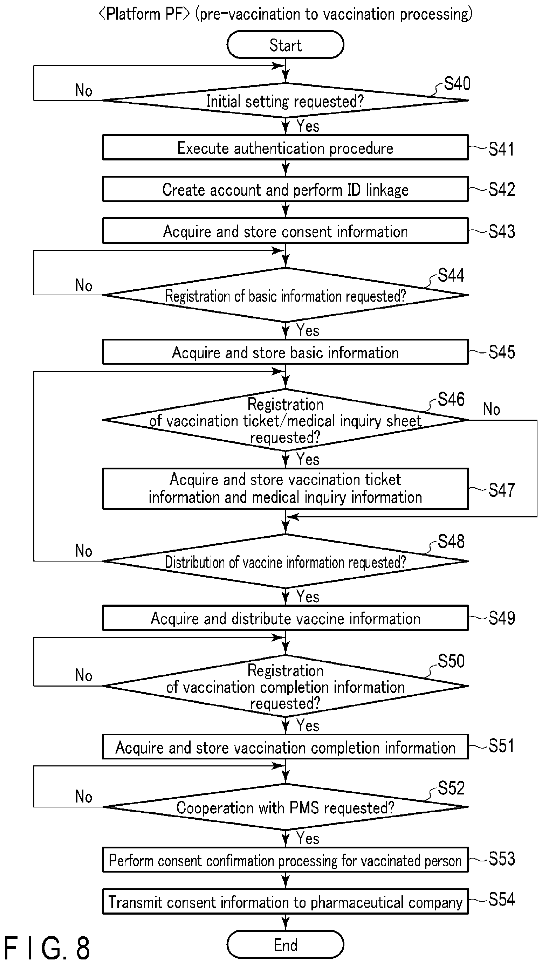

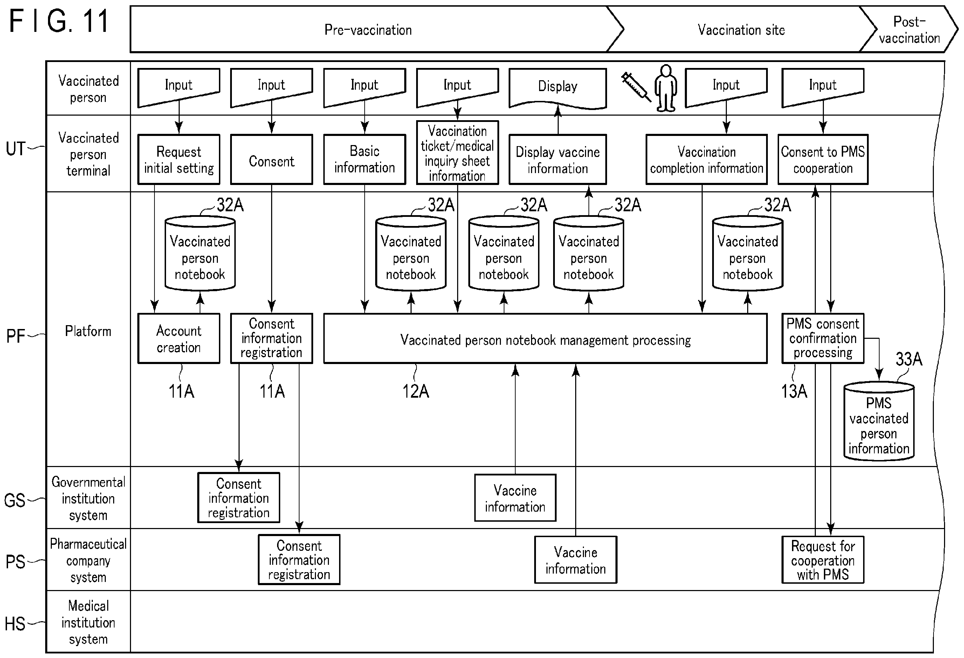

FIG. 6 is a flowchart showing an example of procedures and details of pre-vaccination to vaccination processing executed by the control unit 1B of the vaccinated person terminal UT. FIG. 8 is a flowchart showing an example of procedures and details of pre-vaccination to vaccination processing executed by the control unit 1A of the platform PF. FIG. 11 is a sequence diagram showing a flow of a series of information data in pre-vaccination to vaccination processing among the platform PF, its peripheral systems, and the vaccinated person terminal UT.

### (1-1) Initial Setting of Vaccinated Person Terminal UT

Prior to vaccination, when a person to be vaccinated accesses the platform PF from the vaccinated person terminal UT, initial setting of authentication information and the like is performed between the vaccinated person terminal UT and the platform PF as follows.

That is, as shown in FIG. 6, if an input of a request for initial setting from the person to be vaccinated is detected in step S10, the control unit 1B of the vaccinated person terminal UT requests the initial setting of the account to the platform PF by using the account of the communication carrier or the SNS used by the person, under the control of the authentication/consent request processing section 11B.

In the control unit 1A of the platform PF, if the request for initial setting up of the account is received in step S40 as shown in FIG. 8, under the control of the authentication/consent management processing section 11A, the authentication/consent management processing section 11A first executes the authentication procedure with respect to the vaccinated person terminal UT of the request source in step S41 and stores the authentication information in the authentication/consent-information storage section 31A. Subsequently, in step S42, an account is created for the person to be vaccinated who is the request source to use the vaccinated person notebook of the platform PF. In this account creation processing, the unique identification information (vaccinated person ID) of the person to be vaccinated is issued to the person who is the request source, and a vaccinated-person-notebook storage area exclusively used by the person who is the request source is set in the vaccinated-person-notebook storage section 32A. Then, ID linkage is performed between the vaccinated person ID and the account of the communication carrier or the SNS used by the person to be vaccinated. In the authentication procedure described above, Japanese Public Key Infrastructure (JPKI) may be used for personal authentication of the person to be vaccinated.

Next, a consent procedure is executed between the vaccinated person terminal UT and the platform PF under the control of the authentication/consent request processing section 11B and the authentication/consent management processing section 11A. In the consent procedure, for example, in addition to consent information of the person to be vaccinated with respect to the terms of use of the platform PF, consent information with respect to provision of vaccinated person notebook information or the like of the person to be vaccinated to the pharmaceutical company or the governmental institution, and consent information with respect to distribution of vaccine information or the like by the pharmaceutical company or the governmental institution to the person to be vaccinated are respectively set. Each piece of consent information set in the above consent procedure is stored in the authentication/consent-information storage section 31A in step S43.

Upon completion of the consent procedure, the authentication/consent management processing section 11A of the platform PF sends, to the pharmaceutical company system PS and the governmental institution system GS, information indicating the vaccination target person who has consented to the provision of the vaccinated person notebook information and the like and the distribution of the vaccine information and the like through the communication I/F 4B. The pharmaceutical company and the governmental institution register the information indicating the person to be vaccinated who has consented to the information provision and the information distribution reported from the platform PF in a vaccinated person list.

### (1-2) Registration of Basic Information

After completion of the authentication and consent procedure described above, the person to be vaccinated registers basic information of the person from the vaccinated person terminal UT to the platform PF before vaccination. For example, if an input of a request for registration of the basic information is detected in step S12, the control unit 1B of the vaccinated person terminal UT transmits the basic information input through the input section 62B by the person to be vaccinated to the platform PF in step S13.

On the other hand, if the control unit 1A of the platform PF receives the request for registration of the basic information in step S44, the control unit 1A receives the basic information transmitted from the vaccinated person terminal UT via the communication I/F 4A in step S45 under the control of the vaccinated-person-notebook management processing section 12A, and causes the received basic information to be stored in the vaccinated-person-notebook storage area corresponding to the person to be vaccinated of the request source in the vaccinated-person-notebook storage section 32A. The basic information includes, but is not limited to, information indicating attributes of the person to be vaccinated, for example, the name, age, and contact information such as the postal address, telephone number, and email address.

### (1-3) Registration of Vaccination Ticket/Medical Inquiry Sheet

Subsequently, the person to be vaccinated performs processing of registering written information of the vaccination ticket mailed from the local government or the like before the vaccination and written information of the medical inquiry sheet created by the person him/herself in the vaccinated person notebook of the platform PF.

For example, if a request for registration is detected in step S14 under the control of the vaccinated-person-notebook registration processing section 12B, the control unit 1B of the vaccinated person terminal UT optically reads the information written in the vaccination ticket and the medical inquiry sheet with, for example, the camera 7B in step S15 and decodes the information from the read image data. Then, the decoded information written in the vaccination ticket and the medical inquiry sheet is transmitted from the communication I/F 4B to the platform PF.

In a case where the information described in the vaccination ticket is represented by a QR code (registered trademark), the information written in the vaccination ticket can be easily and accurately input by reading the QR code. In addition, the information written in the medical inquiry sheet may be manually input from the input section 62B of the vaccinated person terminal UT by the person to be vaccinated him/herself, or medical inquiry information created separately may be read from another terminal via a signal cable or a short-range wireless interface such as Bluetooth (registered trademark).

On the other hand, if the control unit 1A of the platform PF receives the request for registration of the vaccination ticket/medical inquiry sheet in step S46, under the control of the vaccinated-person-notebook management processing section 12A, the control unit 1A receives in step S47 the information written in the vaccination ticket and the medical inquiry sheet transmitted from the vaccinated person terminal UT via the communication I/F 4A, and causes the received information to be stored in the vaccinated-person-notebook storage area corresponding to the person to be vaccinated who is the request source in the vaccinated-person-notebook storage section 32A.

In the vaccination ticket, for example, identification information of the person to be vaccinated that is managed by a governmental institution such as a local government, a designated vaccination date and time or period, a vaccination site, a type of a vaccine, and the like are written, but the information is not limited thereto. The medical inquiry sheet includes, but is not limited to, information indicating the body temperature of the person to be vaccinated immediately before the vaccination, whether the person has a chronic disease, the type of a prescribed medication, whether the person has an allergy to a medication, whether the person is pregnant, a change in physical condition in a predetermined period in the past, and the like.

### (1-4) Distribution of Vaccine Information

The pharmaceutical company or the governmental institution distributes a variety of guidance information related to vaccination to the person to be vaccinated who has consented to the information provision and the information distribution. Examples of the information to be distributed include, but are not limited to, guidance information on a vaccine preparation status, a vaccination time, a vaccination site, and precautions such as adverse events.

On the other hand, if the control unit 1A of the platform PF receives a request for distribution of the vaccine information in step S48, under the control of the vaccinated-person-notebook management processing section 12A, the control unit 1A receives the vaccine information transmitted from the pharmaceutical company system PS or the governmental institution system GS, stores the received vaccine information in the vaccinated-person-notebook storage area for the corresponding person to be vaccinated in the vaccinated-person-notebook storage section 32A, and then transfers the vaccine information to the vaccinated person terminal UT of the corresponding person in step S49. As a result, the aforementioned vaccine information is displayed on the display section 61B of the vaccinated person terminal UT, so that the person to be vaccinated can confirm, from the aforementioned vaccine information, precautions relating to the vaccination, the time of the vaccination, the site where the vaccination is scheduled, and the like before the vaccination.

In a case where the person to be vaccinated has consented to send a notification of the address information and the like of the vaccinated person terminal UT to the pharmaceutical company system PS and the governmental institution system GS, the information on the vaccine can be directly distributed from the pharmaceutical company system PS or the governmental institution system GS to the vaccinated person terminal UT.

### (1-5) Registration of Vaccination Completion Information

When the person is vaccinated at a vaccination site such as a medical institution, a vaccination certification seal or a vaccination completion certificate is issued to the vaccinated person from a medical worker or the like. The vaccination certification seal or the vaccination completion certificate includes, for example, the type of the vaccine, the identification information of the pharmaceutical company (manufacturer ID), and the lot number of the vaccine, and may further include a vaccinated person identification number managed by the local government or the like, the date and time of the vaccination, the name of the vaccination site, and the like.

The vaccinated person inputs the information written in the vaccination certification seal or the vaccination completion certificate to his/her own vaccinated person terminal UT. As the input means, for example, optical reading by the camera 7B is used. That is, if a request for reading the vaccination certification seal or the vaccination completion certificate is detected in step S16, under the control of the vaccinated-person-notebook registration processing section 12B, the control unit 1B of the vaccinated person terminal UT activates the camera 7B in step S17 to optically read the information written in the vaccination certification seal or the vaccination completion certificate. The written information is decoded from the read image data, and the decoded written information is transmitted from the communication I/F 4B to the platform PF as the vaccination completion information. Note that the information written in the vaccination certification seal can also be represented by a QR code, in which case the information written in the vaccination certification seal can be input easily and accurately.

On the other hand, if the control unit 1A of the platform PF receives the request for registration of the vaccination completion information read from the vaccination certification seal or the vaccination completion certificate in step S50, under the control of the vaccinated-person-notebook management processing section 12A, the control unit 1A receives in step S51 the vaccination completion information transmitted from the vaccinated person terminal UT via the communication I/F 4A, and causes the received vaccination completion information to be stored in the vaccinated-person-notebook storage area corresponding to the vaccinated person in the vaccinated-person-notebook storage section 32A.

The vaccination completion information stored in the vaccinated-person-notebook storage section 32A is reported to the pharmaceutical company system PS or the governmental institution system GS from the platform PF. At that time, the manufacturer of the vaccine that the person has been vaccinated with is selected, and the vaccination completion information is reported only to the selected pharmaceutical company system PS.

### (1-6) Confirmation of Consent to Request for Cooperation with PMS

The pharmaceutical company transmits a request for cooperation with the PMS from the pharmaceutical company system PS to the vaccinated person. If the request for cooperation with the PMS is received in step S52, under the control of the PMS consent confirmation processing section 13A, the control unit 1A of the platform PF performs consent confirmation processing for the vaccinated person with respect to the request for cooperation with the PMS in step S53.

For example, the PMS consent confirmation processing section 13A transfers a message of the request for cooperation with the PMS to the corresponding vaccinated person terminal UT. On the other hand, if the control unit 1B of the vaccinated person terminal UT receives the request for cooperation with the PMS in step S18 under the control of the PMS consent registration processing section 13B, the control unit 1B causes the received message of the request for cooperation with the PMS to be displayed on the display section 61B. In this state, when the vaccinated person inputs the consent to the request for cooperation with the PMS from the input section 62B, the PMS consent registration processing section 13B confirms in step S19 whether the consent is obtained, and if the consent is obtained, the consent information for the cooperation with the PMS is returned from the communication I/F 4B to the platform PF in step S20. Upon receipt of the consent information with respect to the cooperation with the PMS, the PMS consent confirmation processing section 13A of the platform PF transfers the consent information to the pharmaceutical company system PS of the request source in step S54.

### (2) Post-vaccination Processing

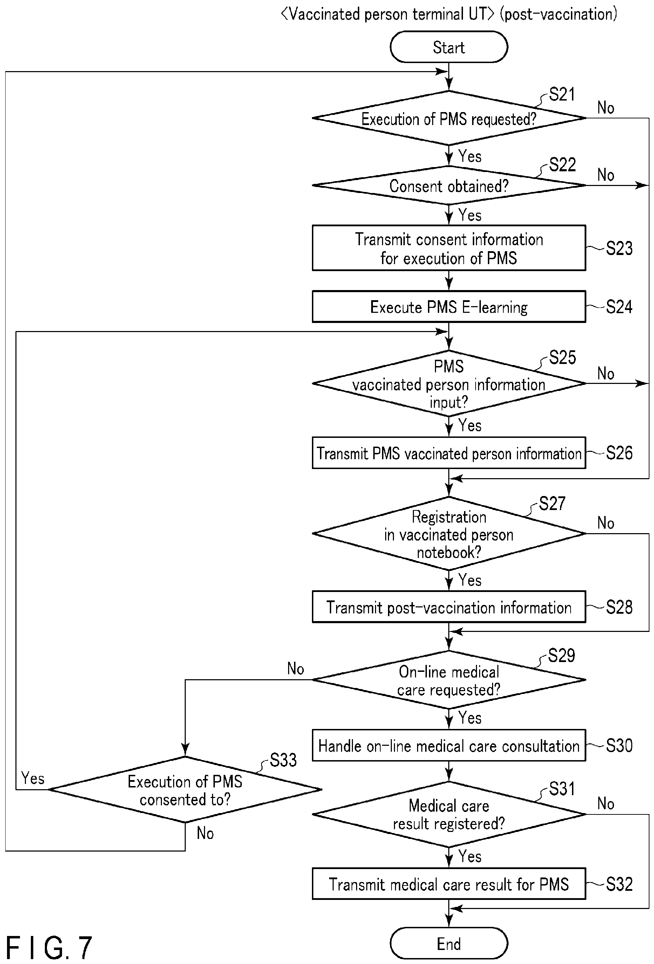

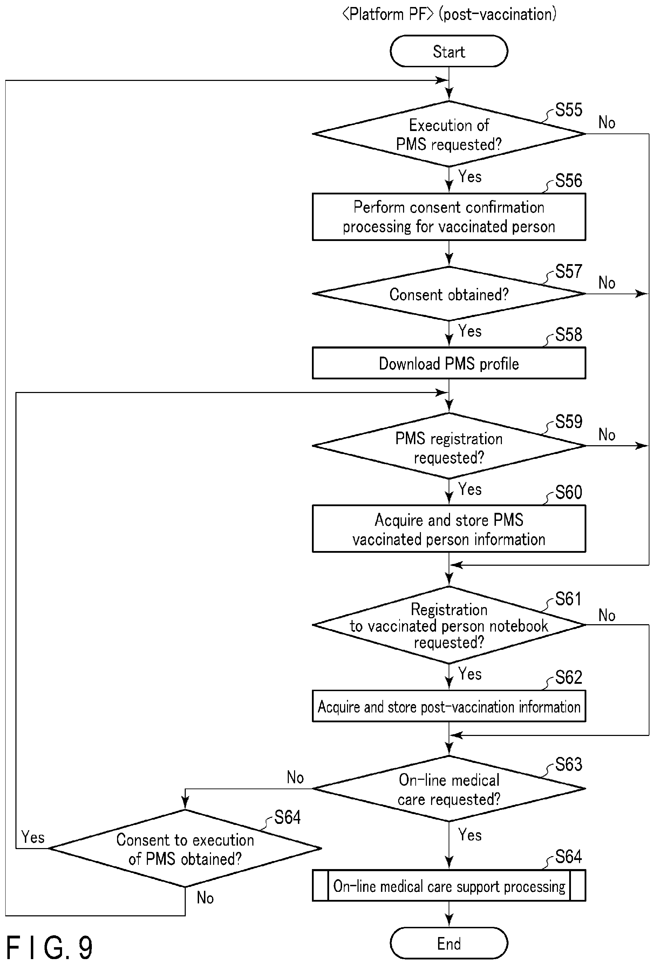

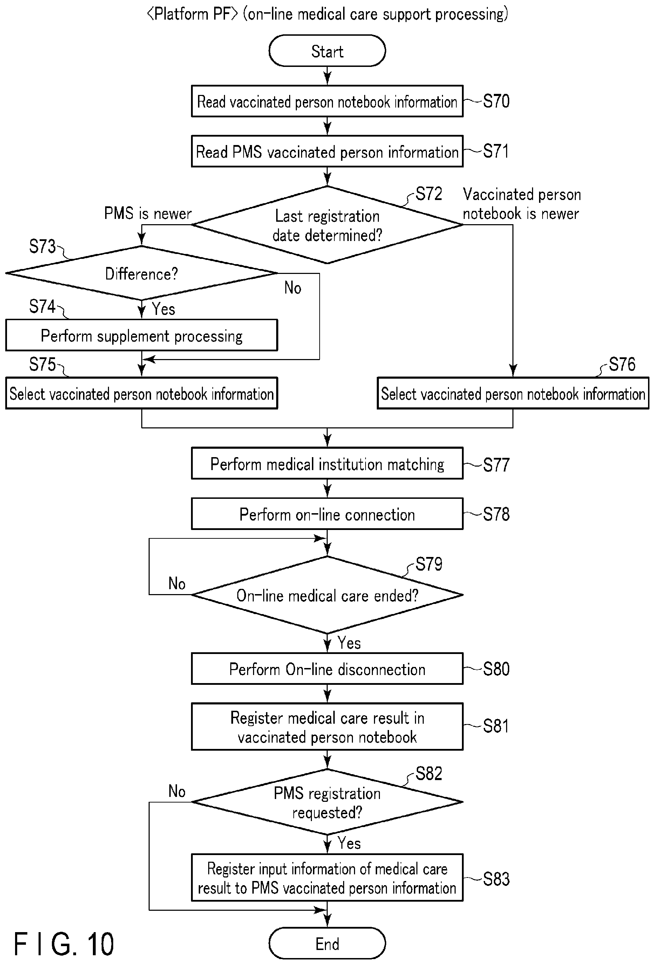

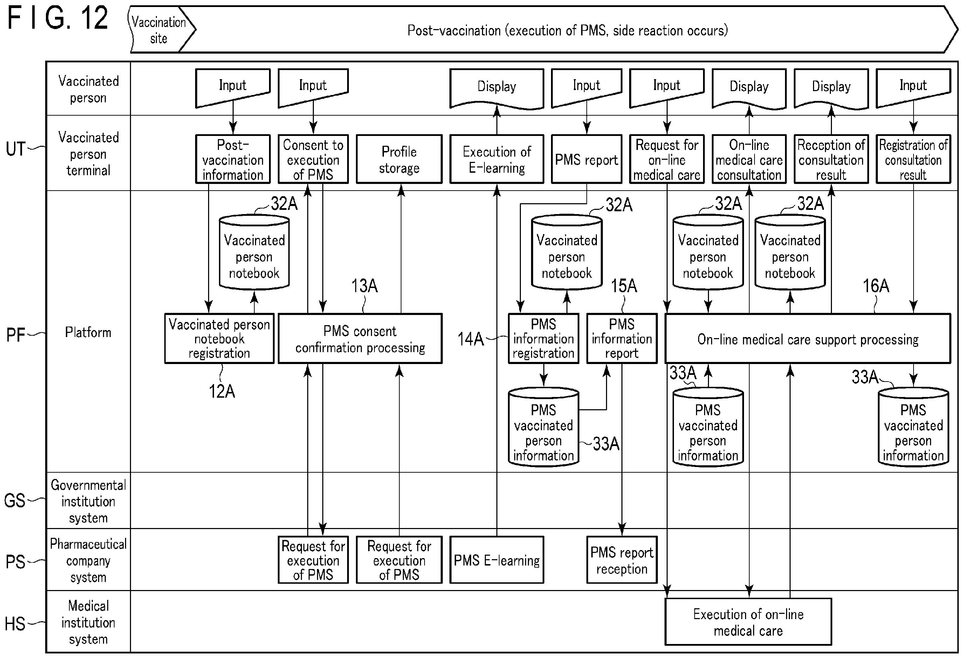

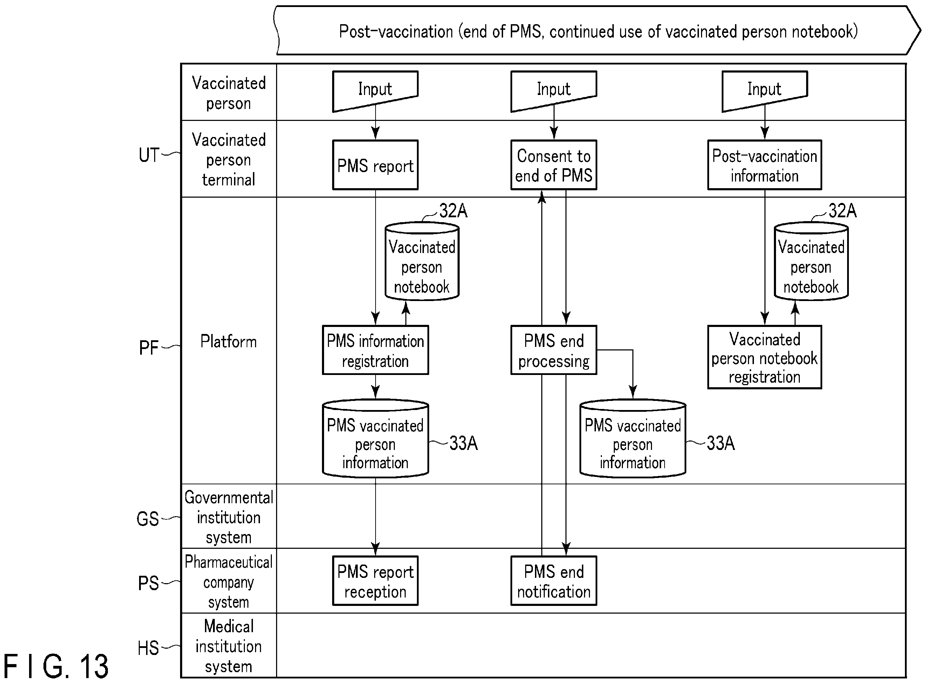

FIG. 7 is a flowchart showing procedures and details of post-vaccination processing by the control unit 1B of the vaccinated person terminal UT, and FIGS. 9 and 10 are flowcharts showing procedures and details of post-vaccination processing by the control unit 1A of the platform PF. FIGS. 12 and 13 are sequence diagrams showing a flow of a series of information data after vaccination among the platform PF, its peripheral systems, and the vaccinated person terminal UT.

### (2-1) Registration of Post-vaccination Information in Vaccinated Person Notebook

The vaccinated person can register information representing his/her physical condition or the like after the vaccination in a discretionary format and at any time in the vaccinated person notebook of the platform PF.

For example, if the vaccinated person inputs a request for registration to the vaccinated person notebook, the control unit 1B of the vaccinated person terminal UT detects the input of the request for registration in step S27. Then, under the control of the vaccinated-person-notebook registration processing section 12B, in step S28, the information representing the physical condition or the like of the vaccinated person after the vaccination input by the person him/herself in a discretionary format at the input section 62B is fetched via the input/output I/F 5B. Next, the fetched information representing the physical condition or the like after the vaccination is transmitted from the communication I/F 4B to the platform PF together with the time information at that time point as post-vaccination information to be registered in the vaccinated person notebook. In a case where the vaccinated person terminal UT is provided with a position measuring function using a GPS sensor, current position information of the vaccinated person terminal UT may be included in the post-vaccination information.

The post-vaccination information is information in which a change in physical condition or the like at each time is input by the vaccinated person in a discretionary format, but may be information in which a corresponding one is selected from a plurality of types of physical conditions prepared in advance for an inexperienced vaccinated person, or may be information input as free text. The registration timing can also be discretionarily selected. For example, each time the person feels a change in physical condition, details of the change can be registered.

On the other hand, if the control unit 1A of the platform PF receives the request for registration of the post-vaccination information to the vaccinated person notebook described above from the vaccinated person terminal UT in step S61, the control unit 1A receives the post-vaccination information transmitted from the vaccinated person terminal UT via the communication I/F 4A in step S62, under the control of the vaccinated-person-notebook management processing section 12A. Then, the control unit 1A causes the received post-vaccination information to be stored, for example, in chronological order, in the vaccinated-person-notebook storage area corresponding to the vaccinated person of the request source in the vaccinated-person-notebook storage section 32A.

### (2-2) Consent Confirmation to Request for Execution of PMS

The pharmaceutical company transmits a request for execution of the PMS from the pharmaceutical company system PS to the vaccinated person who has previously consented to the request for cooperation with the PMS. If the request for execution of the PMS is received in step S55, under the control of the PMS consent confirmation processing section 13A, the control unit 1A of the platform PF performs in step S56 the consent confirmation processing for the vaccinated person with respect to the request for execution of the PMS.

For example, the PMS consent confirmation processing section 13A transfers a message of the request for execution of the PMS to the corresponding vaccinated person terminal UT. On the other hand, if the control unit 1B of the vaccinated person terminal UT receives the request for execution of the PMS in step S21 under the control of the PMS consent registration processing section 13B, the control unit 1B causes the received message of the request for execution of the PMS to be displayed on the display section 61B. In this state, when the vaccinated person inputs the consent to the request for execution of the PMS from the input section 62B, the PMS consent registration processing section 13B confirms in step S22 whether consent is obtained, and if the consent is obtained, the consent information for the execution of the PMS is returned from the communication I/F 4B to the platform PF in step S23.

On the other hand, if the PMS consent confirmation processing section 13A of the platform PF confirms the consent based on the consent information for the execution of the PMS in step S57, the PMS consent confirmation processing section 13A sends a notification of the consent confirmation information to the pharmaceutical company system PS of the request source. When a PMS profile is sent from the pharmaceutical company system PS, the PMS profile is downloaded to the vaccinated person terminal UT in step S58.

Subsequently, a PMS E-learning program is downloaded from the pharmaceutical company system PS to the vaccinated person terminal UT. In step S24, the vaccinated person terminal UT executes the PMS E-learning program. Thus, the vaccinated person can confirm a reporting method with respect to the PMS, etc.

### (2-3) Registration of PMS Vaccinated Person Information

In accordance with the downloaded PMS profile, the control unit 1B of the vaccinated person terminal UT causes the display section 61B to display a message of the request for execution of the PMS and a list of PMS report items for the vaccinated person, for example, at a designated report timing after the vaccination. In this state, for example, it is assumed that the vaccinated person inputs report information indicating his/her physical condition or the like with respect to the displayed report items through the input section 62B. Then, under the control of the PMS vaccinated-person-information registration processing section 14B, in step S25, the control unit 1B of the vaccinated person terminal UT fetches the input PMS report information via the input/output I/F 5B, and transmits the fetched PMS report information together with the time information at this time point as PMS vaccinated person information from the communication I/F 4B to the platform PF.

On the other hand, if the control unit 1A of the platform PF receives a request for PMS registration from the vaccinated person terminal UT, the control unit 1A proceeds from step S59 to step S60. Then, under the control of the PMS vaccinated-person-information management processing section 14A, the PMS vaccinated person information is received via the communication I/F 4A, and the received PMS vaccinated person information is stored in the PMS vaccinated-person-information storage section 33B in association with the vaccinated person ID. At the same time, in order to reflect the PMS vaccinated person information in the vaccinated person notebook, the PMS vaccinated person information is stored in the vaccinated-person-notebook storage area corresponding to the vaccinated person in the vaccinated-person-notebook storage section 32A.

Then, the control unit 1A of the platform PF transmits the PMS vaccinated person information stored in the PMS vaccinated-person-information storage section 33B from the communication I/F 4A to the pharmaceutical company system PS of the request source under the control of the PMS vaccinated-person-information report processing section 15A. In this case, the PMS vaccinated-person-information report processing section 15A may transmit the PMS vaccinated person information to the pharmaceutical company system PS each time the PMS vaccinated person information is acquired, or may transmit the acquired PMS vaccinated person information to the pharmaceutical company system PS in a case where the PMS vaccinated person information includes information indicating that a predetermined change in condition of the vaccinated person is suspected.

### (2-4) Handling Processing for On-line Medical Care

The on-line medical care includes a health care consultation and an on-line medical care consultation. In the health care consultation, the control unit 1A of the platform PF handles the consultation for the vaccinated person under the control of the on-line medical care support processing section 16A, for example, by a chat system. The vaccinated person terminal UT may be connected to a terminal of a medical institution or a health-care center to contact a person in charge of the handling.

On the other hand, in the case of an on-line medical care consultation, the platform PF refers to at least one of the vaccinated person notebook information and the PMS vaccinated person information corresponding to the vaccinated person of the request source, performs matching between the vaccinated person and a plurality of candidate medical institutions, and selects a medical institution capable of handling the consultation. Then, the vaccinated person notebook information or the PMS vaccinated person information corresponding to the vaccinated person is transferred to the selected medical institution system HS, and the medical institution and the vaccinated person terminal UT are connected by a communication link for on-line medical care to enable on-line medical care.

Hereinafter, an example of processing in a case where an on-line medical care consultation is conducted will be described in detail. If the input of a request for the on-line medical care consultation is detected in step S29, the control unit 1B of the vaccinated person terminal UT transmits the request for the on-line medical care consultation to the platform PF in step S30 under the control of the on-line medical care request/response processing section 15B and thereafter starts a series of handling processing for the on-line medical care consultation.

On the other hand, if the control unit 1A of the platform PF receives the request for the on-line medical care consultation from the vaccinated person terminal UT in step S63, the process proceeds to step S74, and an on-line medical care support processing is executed as follows under the control of the on-line medical care support processing section 16A.

FIG. 10 is a flowchart illustrating an example of procedures and details of the processing of the on-line medical care support processing.

In general, registration of post-vaccination information to the vaccinated person notebook is performed relatively frequently by the vaccinated person at a discretionary timing. However, registration of the PMS vaccinated person information is performed at a predetermined timing after the vaccination specified by the pharmaceutical company, for example, at a timing or during a period when a side reaction or the like is highly likely to occur after the vaccination; therefore, the number of registrations tends to be small, and the time interval between registrations tends to be large.

Under these circumstances, the control unit 1A of the platform PF first reads the vaccinated person notebook information and the PMS vaccinated person information corresponding to the vaccinated person of the request source in steps S70 and S71, respectively, under the control of the on-line medical care support processing section 16A. Then, in step S72, the on-line medical care support processing section 16A compares the last registration date and time of the vaccinated person notebook information with the last registration date and time of the PMS vaccinated person information. As a result of the comparison, if the last registration date and time of the vaccinated person notebook information is newer, the process proceeds to step S76 to select the vaccinated person notebook information as it is.

On the other hand, it is assumed that the last registration date and time of the PMS vaccinated person information is newer. In this case, the on-line medical care support processing section 16A proceeds to step S73 and determines whether or not there is a difference between the vaccinated person notebook information and the PMS vaccinated person information. If the PMS vaccinated person information includes information which has not been reflected in the vaccinated person notebook information, the processing of supplementing the vaccinated person notebook information with the PMS vaccinated person information is performed in step S74, and then the vaccinated person notebook information after the supplementing processing is selected in step S75. If there is no difference, the vaccinated person notebook information mentioned above is selected as it is. The PMS vaccinated person information may be selected as it is in addition to the vaccinated person notebook information, although the amount of information increases.

Next, in step S77, the on-line medical care support processing section 16A performs matching between the vaccinated person and a plurality of medical institutions capable of on-line medical care, and selects a medical institution (doctor) satisfying a condition desired by the vaccinated person, for example, a waiting time or a geographical condition in case of face-to-face medical care. In the matching process, for example, the type and severity of a symptom may be taken into consideration by referring to the contents of the selected vaccinated person notebook information or PMS vaccinated person information.

When the medical institution is selected, the on-line medical care support processing section 16A transfers the vaccinated person notebook information to the medical institution. Then, in step S78, the system HS of the medical institution or the doctor terminal thereof and the vaccinated person terminal UT of the request source are connected by a communication link capable of on-line medical care. Thus, image data and voice data for on-line medical care can be transmitted thereafter between the doctor terminal and the vaccinated person terminal UT.

During the on-line medical care, the on-line medical care support processing section 16A monitors whether the on-line medical care ends in step S79. If the end is detected, the communication link is disconnected in step S80.

Subsequently, in step S81, the on-line medical care support processing section 16A receives information indicating a medical care result sent from the doctor terminal, for example, information described in an electronic medical record. Then, the information indicating the received medical care result is added to the corresponding vaccinated person notebook information and transmitted to the vaccinated person terminal UT.

Upon receipt of the information indicating the medical care result, the control unit 1B of the vaccinated person terminal UT stores the received information indicating the medical care result in the data storage unit 3B and causes the received information to be displayed on the display section 61B under the control of the on-line medical care request/response processing section 15B. In this state, if the vaccinated person performs a request operation for registering the information indicating the medical care result in the PMS vaccinated person information and this operation is detected in step S31, the on-line medical care request/response processing section 15B transmits the information indicating the medical care result from the communication I/F 4B to the platform PF in step S32.

On the other hand, if the control unit 1A of the platform PF receives the request for PMS registration sent from the vaccinated person terminal UT in step S82, the control unit 1A receives the information indicating the medical care result via the communication I/F 4A and adds the received information indicating the medical care result to the PMS vaccinated-person-information storage section 33A in step S83 under the control of the on-line medical care support processing section 16A.

### (3) End of PMS Registration after Vaccination

The pharmaceutical company requests that the platform PF end the processing for PMS registration when the pharmaceutical company determines that the follow-up research for the vaccine is no longer necessary after the vaccination, for example, when a period in which side reactions or the like appear has elapsed. In this case, the platform PF executes the following processing.

FIG. 13 is a sequence diagram showing a flow of information data among the platform PF, the pharmaceutical company system PS, and the vaccinated person terminal UT in a case where the PMS registration is ended.

That is, when the control unit 1A of the platform PF receives the request for ending the PMS registration from the pharmaceutical company system PS, the control unit 1A transfers the request for ending the PMS registration to the vaccinated person terminal UT under the control of the PMS consent confirmation processing section 13A.

On the other hand, if the consent information with respect to the end of the PMS registration is returned from the vaccinated person terminal UT, the PMS consent confirmation processing section 13A of the platform PF stores the consent information in the authentication/consent-information storage section 31A and then returns it to the pharmaceutical company system PS. Finally, the PMS vaccinated person information stored in a PMS storage area for the corresponding vaccinated person in the PMS vaccinated-person-information storage section 33A is erased.

The control unit 1A of the platform PF continues the registration processing for the information indicating the change in the physical condition and the like of the vaccinated person in the vaccinated person notebook even after the PMS registration is ended.

### (Functions and Effects)

As described above, in the first embodiment, the platform PF is provided with the vaccinated-person-notebook storage section 32A and the PMS vaccinated-person-information storage section 33A, and the discretionary information indicating the physical condition of the vaccinated person after the vaccination and the information for a PMS report transmitted from the vaccinated person terminal UT are registered in the storage sections 32A and 33A, respectively. By selectively using the vaccinated person notebook information and the PMS vaccinated person information, information provision for the pharmaceutical company and the on-line medical care support are executed.

Therefore, for example, even for a vaccinated person whose medical record or the like is not managed in a medical institution or the like, information after medication can be collected and managed by the platform PF using the vaccinated person terminal UT. Therefore, it is possible to efficiently manage the condition of a wider range of vaccinated persons after vaccination. Further, by selectively using the vaccinated person notebook information and the PMS report information for the pharmaceutical company to deal with the change in the post-medication condition of the vaccinated person, it is possible to perform appropriate handling processing in consideration of the natures of both pieces of information.

In addition, according to the first embodiment, since the consent confirmation procedure of the vaccinated person with respect to the provision of the PMS report information for the pharmaceutical company is automatically executed in advance, the provision processing of the PMS report information can be smoothly performed.

Furthermore, in response to a request for on-line medical care from the vaccinated person terminal UT, the registration date and time of the vaccinated person notebook information and the registration date and time of the PMS vaccinated person information are compared, and if the vaccinated person notebook information is newer, the vaccinated person notebook information is provided to the medical institution as it is, and if the PMS vaccinated person information is newer, the vaccinated person notebook information to which different information of the PMS vaccinated person information not included in the vaccinated person notebook information is added is provided to the medical institution. In this way, it is possible to provide the medical institution with the vaccinated person notebook information including the latest information at all times during the on-line medical care.

Further, when a medical institution for on-line medical care is selected, it is possible to select an optimum medical institution corresponding to the condition of the vaccinated person by taking into account the contents of the vaccinated person notebook information and the PMS vaccinated person information.

### (Other Embodiments)

(1) In the embodiment described above, in the on-line medical care support processing, the platform PF connects the vaccinated person and the doctor terminal of the medical institution via the communication link on a one-to-one basis to perform the on-line medical care. However, the present invention is not limited to this, and in addition to the vaccinated person terminal UT and the doctor terminal, a management terminal of the pharmaceutical company which is a manager of the PMS vaccinated person information may be connected to the communication link to perform communication related to on-line medical care by the three parties. In this way, it is possible for the person in charge in the pharmaceutical company to grasp in real time the symptoms of suspected harmfulness such as side reactions that have appeared in the vaccinated person.
(2) In the embodiment described above, the PMS vaccinated person information is transferred from the platform PF to the pharmaceutical company system PS as it is. However, for example, by providing the platform PF with a processing function for analyzing the PMS vaccinated person information, an event suspected of being harmful such as a side reaction may be estimated from the PMS vaccinated person information, and the estimation result may be reported to the pharmaceutical company. In this case, the information indicating the event suspected of being harmful may also be reported to the governmental institution system GS.
(3) In the embodiment described above, the case of vaccination has been described as an example. However, the present invention is not limited to this case and can be applied to a case where a pharmaceutical company or the like conducts clinical trials in the course of development of a new medication or the like other than a vaccine. That is, the type of medication is not limited to a vaccine, and other medications may be used.
(4) In addition, the functional configuration of the platform PF and the procedures and details of the processing executed by these functions, the type of the handling processing that selectively uses the vaccinated person notebook information and the PMS vaccinated person information and the procedure for authentication and consent confirmation, the type and format of the information registered in the vaccinated person notebook, the format of the information and the registration request timing for the PMS registration, and the like can be variously modified without departing from the scope of the present invention.

In addition to the application to the PMS and clinical trials, for example, in the utilization form of the on-line medical care by the three parties as described above, it is conceivable for a pharmacist terminal to be added in place of the management terminal of the pharmaceutical company and used as a medication treatment support tool when performing medical-pharmaceutical cooperative medical care in which a doctor in charge of medication treatment and a pharmacist performing medication instructions cooperate with each other to treat a patient suffering from diseases requiring continuous medical care at home.

The program concerning the present embodiment may be transferred in a state of being stored in an electronic device or in a state of not being stored in an electronic device. In the latter case, the program may be transferred through a network or may be transferred in a state of being stored in a storage medium. The storage medium is a non-transitory tangible medium. The storage medium is a computer-readable medium. The storage medium may be of any type, such as a CD-ROM or a memory card, as long as it can store programs and can be read by the computer.

Although the embodiments of the present invention have been described in detail in the foregoing, the description is merely an example of the present invention in all of its aspects. Various improvements and modifications can be made without departing from the scope of the present invention. In other words, a specific configuration according to an embodiment may be adopted as appropriate when implementing the present invention.

In short, the present invention should not be limited to the above-described embodiments as-is, but may be embodied by modifying the components without departing from the scope of the invention at the implementation stage. In addition, various inventions can be made by suitably combining the structural elements disclosed in connection with the above embodiments. For example, some structural elements may be deleted from all the structural elements described in the embodiments. Furthermore, structural elements over different embodiments may be appropriately combined.

### REFERENCE SIGNS LIST

PF: management control apparatus (platform)
UT1 to UTn: vaccinated person terminals
HS: medical institution system
PS: pharmaceutical company system
GS: governmental institution system
NW: network
1A, 1B: control unit
2A, 2B: program storage unit
3A, 3B: data storage unit
4A, 4B: communication I/F
5B: input/output I/F
6B: input/output device
7B: camera
11A: authentication/consent management processing section
12A: vaccinated-person-notebook management processing section
13A: PMS consent confirmation processing section
14A: PMS vaccinated-person-information management processing section
15A: PMS vaccinated-person-information report processing section
16A: on-line medical care support processing section 11B: authentication/consent request processing section
12B: vaccinated-person-notebook registration processing section
13B: PMS consent registration processing section
14B: PMS vaccinated-person-information registration processing section
15B: on-line medical care request/response processing section
31A: authentication/consent-information storage section
32A: vaccinated-person-notebook storage section
33A: PMS vaccinated-person-information storage section

## Claims

1. A medication target person management system for managing a post-medication condition of a medication target person, the medication target person management system comprising a terminal device configured to be used by the medication target person and a management control apparatus capable of communicating information data with the terminal device via a network,
the terminal device comprising:
a first transmission processing section configured to transmit, to the management control apparatus via the network, first medication management information including the post-medication condition of the medication target person input for a discretionary item; and
a second transmission processing section configured to transmit, to the management control apparatus via the network, second medication management information including the post-medication condition of the medication target person input for a report item designated in advance;
the management control apparatus comprising:
a first storage section in which a storage area for storing medicated person notebook information corresponding to the medication target person is set;
a second storage section in which a storage area for storing report information for a medication manager corresponding to the medication target person is set;
a first acquisition processing section configured to acquire the first medication management information from the terminal device via the network, associate the acquired first medication management information with identification information of the medication target person, and cause the first storage section to store the first medication management information as the medicated person notebook information;
a second acquisition processing section configured to acquire the second medication management information from the terminal device via the network, associate the acquired second medication management information with the identification information of the medication target person, and cause the second storage section to store the second medication management information as the report information; and
a post-medication handling processing section configured to selectively use the stored first medication management information and the stored second medication management information to execute handling processing according to a change in the post-medication condition for the medication target person.

2. A management control apparatus for managing a post-medication condition of a medication target person and capable of communicating information data with a terminal device configured to be used by the medication target person via a network, the management control apparatus comprising:
a first storage section in which a storage area for storing medicated person notebook information corresponding to the medication target person is set;
a second storage section in which a storage area for storing report information for a medication manager corresponding to the medication target person is set;
a first acquisition processing section configured to acquire, from the terminal device via the network, first medication management information prepared for a discretionary item and including the post-medication condition of the medication target person, associate the acquired first medication management information with identification information of the medication target person, and cause the first storage section to store the first medication management information as the medicated person notebook information;
a second acquisition processing section configured to acquire, from the terminal device via the network, second medication management information prepared for a report item designated in advance and including the post-medication condition of the medication target person, associate the acquired second medication management information with the identification information of the medication target person, and cause the second storage section to store the second medication management information as the report information; and
a post-medication handling processing section configured to selectively use the stored first medication management information and the stored second medication management information to execute handling processing according to a change in the post-medication condition for the medication target person.

3. The management control apparatus according to claim 2, further comprising a consent confirmation processing section configured to confirm whether or not the medication target person consents to report the second medication management information,
wherein the second storage section sets a storage area for the report information corresponding to the medication target person who has consented to report the second medication management information among medication target persons.

4. The management control apparatus according to claim 2, wherein, if the second medication management information is newly acquired or if the acquired second medication management information includes a content indicating that a predetermined change in condition of the medication target person is suspected, the post-medication handling processing section performs processing of transferring the acquired second medication management information to the medication manager.

5. The management control apparatus according to claim 2, wherein the post-medication handling processing section, in a case of receiving a request for on-line medical care consultation by the medication target person from the terminal device, performs processing of selectively transferring the first medication management information and the second medication management information corresponding to the medication target person to a medical institution and setting a communication link for on-line medical care between the terminal device and the medical institution.

6. The management control apparatus according to claim 5, wherein the post-medication handling processing section, in a case of receiving a request for on-line medical care consultation by the medication target person from the terminal device, selects the medical institution based on the first medication management information and the second medication management information corresponding to the medication target person.

7. The management control apparatus according to claim 5, wherein the post-medication handling processing section, in a case of receiving a request for on-line medical care consultation by the medication target person from the terminal device, selects medication management information based on respective registration dates and times of the first medication management information and the second medication management information corresponding to the medication target person, and transfers the selected medication management information to the medical institution.

8. The management control apparatus according to claim 5, wherein the post-medication handling processing section, in a case of receiving a request for on-line medical care consultation by the medication target person from the terminal device, generates third medication management information obtained by adding a difference between the first medication management information and the second medication management information to the first medication management information corresponding to the medication target person, and transfers the generated third medication management information to the medical institution.

9. The management control apparatus according to claim 5, wherein the post-medication handling processing section performs processing of acquiring information indicating a medical care result of the on-line medical care from the medical institution and additionally registering the acquired information indicating the medical care result to the corresponding first medication management information.

10. A terminal device configured to be used by the medication target person and capable of communicating information data with the management control apparatus according to claim 2 via the network, the terminal device comprising:
a first transmission processing section configured to transmit, to the management control apparatus via the network, the first medication management information including the post-medication condition of the medication target person input for a discretionary item; and
a second transmission processing section configured to transmit, to the management control apparatus via the network, the second medication management information including the post-medication condition of the medication target person input for a report item designated in advance.

11. A medication target person management method executed by a system including a terminal device configured to be used by a medication target person and a management control apparatus capable of communicating information data with the terminal device via a network, the management control apparatus comprising a first storage section in which a storage area for storing medicated person notebook information corresponding to the medication target person is set, and a second storage section in which a storage area for storing report information for a medication manager corresponding to the medication target person is set, the medication target person management method comprising:
executing, by the terminal device:
transmitting, to the management control apparatus via the network, first medication management information including the post-medication condition of the medication target person input for a discretionary item; and
transmitting, to the management control apparatus via the network, second medication management information including the post-medication condition of the medication target person input for a report item designated in advance,
executing, by the management control apparatus:
acquiring the first medication management information from the terminal device via the network and causing the first storage section to store the acquired first medication management information as the medicated person notebook information in association with identification information of the medication target person;
acquiring the second medication management information from the terminal device via the network and causing the second storage section to store the acquired second medication management information as the report information in association with the identification information of the medication target person; and
selectively using the stored first medication management information and the stored second medication management information to execute handling processing according to a change in the post-medication condition for the medication target person.

12. A program storage medium storing a program for causing a hardware processor included in the management control apparatus according to any one of claims 2 to 9 to execute processing of each of processing sections included in the management control apparatus.

13. A program storage medium storing a program for causing a hardware processor included in the terminal device according to claim 10 to execute processing of each of the processing sections included in the terminal device.
